(19) Europäisches Patentamt
European Patent Office
Office européen des brevets

(11) Publication number: **0 166 357**
**A2**

(12) **EUROPEAN PATENT APPLICATION**

(21) Application number: 85107503.6

(22) Date of filing: 18.06.85

(51) Int. Cl.[4]: C 07 K 5/00, C 07 K 5/06, C 07 D 223/16, A 61 K 37/02

(30) Priority: 26.06.84 US 624856
01.04.85 US 718597

(43) Date of publication of application: 02.01.86 Bulletin 86/1

(84) Designated Contracting States: CH DE FR GB IT LI NL

(71) Applicant: MERCK & CO. INC., 126, East Lincoln Avenue P.O. Box 2000, Rahway New Jersey 07065 (US)

(72) Inventor: Parsons, William H., 2171 Oliver Street, Rahway, N.J. 07065 (US)
Inventor: Patchett, Arthur A., 1090 Minisink Avenue, Westfield, N.J. 07090 (US)

(74) Representative: Blum, Rudolf Emil Ernst et al, c/o E. Blum & Co Patentanwälte Vorderberg 11, CH-8044 Zürich (CH)

(54) **Benzofused lactams and pharmaceutical compositions containing them.**

(57) Benzofused lactams and salts thereof are disclosed. They are used as active ingredient of pharmaceutical compositions which are useful as cholecystokinin antagonists.

## TITLE OF THE INVENTION

Benzofused lactams and pharmaceutical compositions containing them.

## BACKGROUND OF THE INVENTION

Cholecystokinin (CCK) is a neuropeptide composed of thirty-three aminoacids. [See: Mutt and Jorpes, Biochem. J. 125 678 (1971)]. The carboxyl terminal octapeptide (CCK-8) also occurs naturally and is fully active. CCK exists in both gastrointestinal tissue and the central nervous system. [V. Mutt, Gastrointestinal Hormones, G. B. J. Glass, Ed., Raven Press, N.Y., (1980) p. 169.] CCK is believed to play an important role in appetite regulation and CCK may be a physiological satiety hormone. [G. P. Smith, Eating and Its Disorders, A. J. Stunkard and E. Stellar, Eds, Raven Press, New York, 1984, p. 67.]

Among additional effects of CCK are stimulation of colonic motility, stimulation of gall bladder contraction, stimulation of pancreatic enzyme secretion, and inhibition of gastric emptying. CCK reportedly co-exists with dopamine in certain mid-brain neurons and thus may also play a role in the functioning of dopaminergic systems in the brain as well as serving as a neurotransmitter in its own right. [See: A. J. Prange et al., "Peptides in the Central Nervous System", Ann. Repts. Med. Chem. 17 31, 33 (1982) and references cited therein; J. A. Williams, Biomed. Res. 3, 479 107 (1982); and J. E. Morley, Life Sci. 30 (1982)].

CCK antagonists are useful in the treatment and prevention of CCK-related disorders of the gastrointestinal, central nervous and appetite regulatory systems of animals, especially humans. Three distinct chemical classes of CCK receptor antagonists have been reported. One class comprises derivatives of cyclic nucleotides; detailed structure-function studies have demonstrated that of the various members of this class, dibutyryl cyclic GMP is the most potent. [See; N. Barlos et al., Am. J. Physiol., 242, G161 (1982) and P. Robberecht et al., Mol., Pharmacol., 17, 268 (1980)]. The second class comprises peptide antagonists which are C-terminal fragments and analogs of CCK. Recent structure-function studies have shown that both shorter C-terminal fragments of CCK (Boc-Met-Asp-Phe-$NH_2$, Met-Asp-Phe-$NH_2$) as well as longer CCK fragments (Cbz-Tyr($SO_3H$)-Met-Gly-Trp-Met-Asp-$NH_2$) can function as CCK antagonists. [See: R. T. Jensen et al., Biochim. Biophys. Acta., 757,

250 (1983) and M. Spanarkel et al., J. Biol. Chem., 258, 6746 (1983)]. The third class of CCK receptor antagonists comprises the amino acid derivatives; proglumide, a derivative of glutaramic acid, and the N-acyl tryptophans including para-chlorobenzoyl-L-tryptophan (benzotript). [See W. F. Hahne et al., Proc. Natl. Acad. Sci. U.S.A., 78, 6304 (1981) and R. T. Jensen et al., Biochem. Biophys. Acta., 761, 269 (1983)]. All of these compounds are relatively weak antagonists of CCK ($IC_{50}$:$10^{-4}$-$10^{-6}$M).

SUMMARY OF THE INVENTION

It has now been found that compounds of this invention are antagonists of cholecystokinin (CCK). These CCK antagonists are useful in the treatment and prevention of CCK-related disorders of the gastrointestinal, central nervous and appetite regulatory systems of mammals, especially humans.

DETAILED DESCRIPTION OF THE INVENTION

The compounds of this invention are benzofused lactams of the formula:

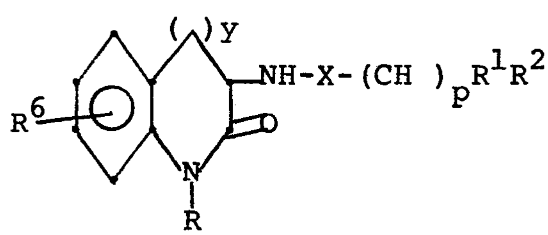

1

wherein:

X is absent or carbonyl;

R is lower $C_1$-$C_8$alkyl;

$C_6$ or $C_{10}$-aryllower $C_1$-$C_8$alkyl;

Dr.IM.-vd
17.5.1985

EU 1249

lower $C_1$-$C_4$alkyl containing a carbonyl group which carbonyl group can be substituted with $C_1$-$C_4$alkyl, hydroxy, $C_1$-$C_4$alkoxy, or $NR^3$ wherein $R^3$ is hydrogen, $C_1$-$C_4$alkyl, carboxyloweralkyl, carboxamidoloweralkyl, arylalkyl wherein the aryl is $C_5$-$C_{10}$ and the alkyl is $C_1$-$C_4$;

$R^1$ and $R^2$ are independently

hydrogen;

lower $C_1$-$C_8$alkyl;

unsubstituted or substituted carbonyl wherein the substituents are alkoxy, alkyl of $C_1$-$C_8$, hydroxyalkyl of $C_1$-$C_8$, amino, loweralkylamino, $C_6$ or $C_{10}$-arylloweralkylamino, carboxyloweralkylamino, carboxamidoloweralkylamino;

$NR^4R^5$ wherein $R^4$ and $R^5$ can independently be unsubstituted or substituted carbonyl, or unsubstituted or substituted carboxyloweralkyl, or unsubstituted or substituted carboxamidoloweralkyl wherein the substituents are $C_1$-$C_4$alkyl,- $C_1$-$C_4$alkyloxy, $C_6$ or $C_{10}$-aryl loweralkyl, $C_6$-$C_{10}$-arylloweralkoxy, aminoloweralkyl, substituted aminoloweralkyl;

substituted loweralkyl wherein the substituents are halo, hydroxy, carboxy, carboxamido, carbonyl, substituted carbonyl wherein the substituent is $C_1$-$C_8$ alkyl-OH, loweralkylthio, loweralkoxy, loweralkoxycarbonyl, lower-$C_6$ or $C_{10}$-arylalkoxycarbonyl, amino, loweralkylamino, lowerdialkylamino, acylamino;

substituted loweralkylamino wherein the substituent can be halo, hydroxy, alkoxy or cyano;

$C_6$ or $C_{10}$-arylloweralkylamino;

$C_6$ or $C_{10}$-aryloxy;

$C_6$ or $C_{10}$-arylthio;

$C_6$ or $C_{10}$-arylalkyloxy;

$C_6$ or $C_{10}$-arylalkylthio;

benzofused cycloalkyl or bicycloalkyl of from 8-12 carbon atoms;

aryl or heteroaryl wherein the aryl is $C_6$ or $C_{10}$ containing an O, N or S heteroatom and which may be mono-, di- or trisubstituted by loweralkyl, hydroxy, loweralkoxy, halo, amino, acylamino, loweralkylthio or aminoloweralkyl;

benzofused cycloalkyl or bicycloalkyl of from 8 to 12 carbon atoms;

$C_6$ or $C_{10}$-arylloweralkyl;

$C_6$ or $C_{10}$-arylloweralkenyl;

heteroloweralkyl and heteroloweralkenyl in which the aryl or heteroaryl rings may be mono-, di- or tri-substituted by halo, loweralkyl, hydroxy, loweralkoxy, amino, loweralkylamino, diloweralkylamino, aminoloweralkyl, acylamino, carboxy, haloloweralkyl, nitro, cyano or sulfonamido;

arylalkyl or heteroarylalkyl wherein the aryl is $C_6$ or $C_{10}$ containing an O, N or S heteroatom and which include branched loweralkyl groups;

substituted arylalkyl or substituted heteroarylalkyl which include branched

loweralkyl groups wherein the loweralkyl groups can be substituted by amino, acylamino, or hydroxyl and the aryl and heteroaryl groups are $C_6$ or $C_{10}$ containing an O, N or S heteroatom and can be substituted by halo, dihalo, loweralkyl, hydroxy, loweralkoxy, $C_6$ or $C_{10}$-aryloxy, $C_6$ or $C_{10}$-aroyl, $C_6$ or $C_{10}$-arylthio, amino, aminoloweralkyl, loweralkanoylamino, $C_6$ or $C_{10}$-aroylamino, lowerdialkylamino, loweralkylamino, hydroxy, hydroxyloweralkyl, trihaloloweralkyl, nitro, cyano, or sulfonamido;

any of the arylloweralkyl or alkenyl and heteroloweralkyl or alkenyl groups described above in which the aryl or heteroaryl ring is partially or completely hydrogenated;

substituted loweralkyl having the formula $R_A^1(CH_2)_n-Q-(CH_2)_m$ wherein n is 0-2, m is 1-3, $R_A^1$ is aryl or heteroaryl wherein the aryl is $C_6$ or $C_{10}$ containing an O, N or S heteroatom optionally substituted by amino, lowerdialkylamino, loweralkylamino, hydroxy, hydroxyloweralkyl, aminoloweralkyl, trihaloloweralkyl, cyano, nitro, sulfonamido, $C_6$-$C_{10}$-aroyl, loweralkyl, halo, dihalo, and loweralkoxy, and Q is O, S, SO, $SO_2$, N-$R_B^1$, CONR$_C^1$, NR$_C^1$CO, CH=CH wherein $R_B^1$ is hydrogen, loweralkyl, $C_6$ or $C_{10}$-aryl, $C_6$ or $C_{10}$-arylalkyl, loweralkanoyl, or $C_6$ or $C_{10}$-aroyl, and

$R^1_C$ is hydrogen, or loweralkyl;

$R^6$ is hydrogen;

halo;

hydroxy;

$C_1$-$C_8$alkyl;

lower$C_1$-$C_8$alkoxy;

y is 1-3;

p is 0-2 with the proviso that when p is O, X is carbonyl; and,

the pharmaceutically acceptable salts thereof.

The straight chain, branched, saturated and unsaturated hydrocarbons recited above are represented by such compounds as methyl, ethyl, n-propyl, i-propyl, n-butyl, i-butyl, t-butyl, n-pentyl, i-pentyl, n-hexyl; or vinyl, allyl, butenyl, and the like.

The loweralkoxy substituent represents a loweralkyl group attached through an oxygen bridge.

The arylalkyl and heteroarylalkyl substituents recited above represent aryl or heteroaryl groups as herein defined attached through a straight or branched chain hydrocarbon of from one to eight carbon atoms such as, for example, benzyl, phenethyl, 3,3-diphenylpropyl, 3-indolylmethyl, and the like.

Halo means chloro, bromo, iodo, or fluoro.

The aryl substituent is illustrated by phenyl, naphthyl, or biphenyl.

The heteroaryl substituent recited above represents any 5- or 6-membered aromatic ring containing from one to three O, N or S heteroatoms such as, for example, pyridyl, thienyl, furyl, imidazolyl, and thiazolyl as well as any bicyclic

group in which any of the above heterocyclic rings is fused to a benzene ring such as, for example, indolyl, quinolinyl, isoquinolinyl, benzimidazolyl, benzothiazolyl, and benzthienyl.

The acylamino substituent represents loweralkanoylamino, arylalkoxycarbonyl, alkyloxycarbonyl, and aroylamino, such as acetylamino, benzyloxycarbonylamino, and benzoylamino.

The Formula 1 compounds can be used in the form of salts derived from inorganic or organic acids and bases. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine and N-methyl-D-glucamine, and salts with amino acids such as arginine, lysine, and so forth. Water or oil-soluble or dispersible products are thereby obtained.

An embodiment of this invention is the use of the compounds of Formula 1 for the treatment and

the prevention of disorders of the gastrointestinal, central nervous, and appetite regulatory systems of mammals, especially of man. Specifically, the Formula 1 compounds are useful in treatment and prevention of disorder of gastric acid secretion, gastrointestinal motility, pancreatic secretions, and dopaminergic functions. The compounds of Formula 1 are especially useful in the prevention and treatment of irritable bowel syndrome.

The ability of the compounds of Formula 1 to antagonize CCK makes these compounds especially useful in the treatment and prevention of disease states wherein CCK may be involved, for example, gastrointestinal disorders such as irritable bowel syndrome, ulcers, excess pancreatic or gastric secretion, acute pancreatis, motility disorders, central nervous system disorders caused by CCK's interaction with dopamine such as neuroleptic disorders, tardive, dyskinesia, Parkinson's disease, psychosis or Gilles de la Tourette Syndrome, and disorders of appetite regulatory systems.

CCK In Vitro Activity of Formula 1 Compounds

The CCK biological activity of the compounds of Formula 1 have been evaluated using an $^{125}I$-CCK receptor binding assay and *in vitro* isolated tissue preparations.

Materials and Methods

1. CCK receptor binding (pancreas)

CCK-33 was radiolabeled with $^{125}I$-Bolton Hunter reagent (2000 Ci/mmole) as described by Sankara et al. [J. Biol. Chem. 254: 9349-9351, (1979)]. Receptor binding was performed according to

Innis and Snyder [Proc. Natl. Acad. Sci. 77: 6917-6921, (1980)] with the minor modification of adding the additional protease inhibitors, phenylmethane sulfonyl fluoride and o-phenanthroline. The latter two compounds have no effect on the $^{125}$I-CCK receptor binding assay.

Male Sprague-Dawley rats (200-350g) were sacrificed by decapitation. The whole pancreas was dissected free of fat tissue and was homogenized in 20 volumes of ice-cold 50 mM, Tris HCl (pH 7.7 at 25°C) with a Brinkman Polytron PT 10. The homogenates were centrifuged at 48,000 g for 10 min. Pellets were resuspended in Tris Buffer, centrifuged as above and resuspended in 200 volumes of binding assay buffer (50 mM Tris HCl, pH 7.7 at 25°C, 5 mM dithiothriethel, 0.1 mM bacitracin, 1.2 mM phenylmethane sulfonyl fluoride and 0.5 mM o-phenanthroline). For the binding assay, 25 μl of buffer (for total binding) or unlabeled CCK-8 sulfate to give a final concentration of 1μM (for nonspecific binding) or the compounds of Formula 1 (for determination inhibition of $^{125}$I-CCK binding) and 25 μl of $^{125}$I-CCK-33 (30,000-40,000 cpm) were added to 450 μl of the membrane suspensions in microfuge tubes. All assays were run in duplicate or triplicate. The reaction mixtures were incubated at 37°C for 30 minutes and centrifuged in a Beckman Microfuge (4 minutes) immediately after adding 1 ml of ice-cold incubation buffer. The supernatant was aspirated and discarded, pellets were counted with a Beckman gamma 5000.

CCK Receptor Binding (Brain)

CCK-33 was radiolabeled with and the binding was performed according to the description for the pancreas method with modifications according to Saito, et al. (J. Neurochem, 37, 483-490 (1981)).

Hall Hartley guinea pigs (300-500 g) were sacrificed by decapitation and the brains were removed and placed in ice-cold 50 mM, Tris HCl plus 7.58 g/l Tritma-7.4 (pH 7.4 at 25°C) Cerebral cortex was dissected and used as a receptor source. Each gram of fresh guinea pig brain tissue was homogenized in 10 ml of Tris/Trizma buffer with a Brinkman polytron PT-10. The homogenates were centrifuged at 42,000 g for 15 minutes. Pellets were resuspended in Tris Buffer, centrifuged as above and resuspended in 200 volumes of binding assay buffer (10 mM HEPES, pH 7.7 at 25°C, 5 mM Mg $Cl_2$, mM EGTA, 0.4% BSA (bovine serum albumin) and 0.25 mg/ml bacitracin). For the binding assay, 25 µl of buffer (for total binding) or unlabeled CCK-8 sulfate to give a final concentration of 1µM (for nonspecific binding) or the compounds of Formula 1 (for determination inhibition of $^{125}$I-CCK binding) and 25 µl of $^{125}$I-CCK-33 (30,00-40,000 cpm) were added to 450 µl of the membrane suspensions in microfuge tubes. All assays were run in duplicate or triplicate. The reaction mixtures were incubated at 25°C for 2 hours and centrifuged in a Beckman Microfuge (4 minutes) immedidately after adding 1 ml of ice-cold incubation buffer. The supernatant was aspirated and discarded, pellets were counted with a Beckman gamma 5000.

In Vitro Results

1. Effect of The Compounds of Formula 1 on $^{125}$I-CCK-33 receptor binding

Compounds of Formula 1 inhibited specific $^{125}$I-CCK-33 binding in a concentration dependent manner with an $IC_{50}$ less than or equal to 100 μM such as, for example:

1-t-butoxycarbonylmethyl-3-(D-N-acetyltryptophanyl)-aminohomodihydrocarbostyril, $IC_{50}$ = 1.6 μM;

1-t-butoxycarbonylmethyl-3-(L-N-acetyltryptophanyl)-aminohomodihydrocarbostyril, $IC_{50}$ = 2.8 μM;

1-t-butoxycarbonylmethyl-3-(L-N-carbobenzyloxy-tryptophanyl)aminohomodihydrocarbostyril, $IC_{50}$ = 1.2 μM;

1-methyl-3-(1-carboethoxy-3-phenyl-1-propyl)amino-homodihydrocarbostyril, $IC_{50}$ = 60 μM; and,

1-carbomethoxymethyl-3-(3-indole methyl)aminohomo-dihydrocarbostyril, $IC_{50}$ = 15 μM.

Thus, in accordance with the present invention, there is provided a pharmaceutical composition for and a method of treating gastrointestinal disorders, central nervous system disorders, or regulating appetite which comprises administering to a patient in need of such treatment a pharmaceutically effective amount of a compound of Formula 1.

For administration, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be

utilized. Whatever the dosage form, it will contain a pharmaceutically effective amount of the compounds of the invention.

The present compositions can be administered orally or other than orally; e.g., parenterally, by insufflation, topically, rectally, etc.; using appropriate dosage forms; e.g., tablets, capsules, suspensions, solutions, and the like, for oral administration; suspension emulsions, and the like, for parenteral administration; solutions for intravenous administration; and ointments, transdermal patches, and the like, for topical administration.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets containing the active ingredient in admixture with non-toxic pharmaceutically acceptable excipients may also be manufactured by known methods. The excipients used may be for example, (1) inert diluents such as calcium carbonate, sodium carbonate, lactose, calcium phosphate or sodium phosphate; (2) granulating and disintegrating agents such as corn starch, or alginic acid; (3) binding agents such as starch, gelatin or acacia, and (4) lubricating agents such as magnesium stearate, stearic acid or talc. The tablets may be uncoated or they may be coated by known techniques to delay disintegration and

absorption in the gastrointestinal tract and thereby provide a sustained action over a longer period. For example, a time delay material such as glyceryl monostearate or glyceryl distearate may be employed. They may also be coated by the techniques described in U.S. Patents 4,256,108; 4,160,452; and 4,265,874 to form osmotic therapeutic tablets for controlled release.

In some cases, formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, for example peanut oil, liquid paraffin, or olive oil.

Aqueous suspensions normally contain the active materials in admixture with excipients suitable for the manufacture of aqueous suspensions. Such excipients may be

(1) suspending agents such as sodium carboxymethylcellulose, methylcellulose, hydroxypropylmethylcellulose, sodium alginate, polyvinylpyrrolidone, gum tragacanth and gum acacia;

(2) dispersing or wetting agents which may be

(a) a naturally-occurring phosphatide such as lecithin,

(b) a condensation product of an alkylene oxide with a fatty acid, for example, polyoxyethylene stearate,

(c) a condensation product of ethylene oxide with a long chain aliphatic alcohol, for example, heptadeca-ethyleneoxycetanol,

(d) a condensation product of ethylene oxide with a partial ester derived from a fatty acid and a hexitol such as polyoxyethylene sorbitol monooleate, or

(e) a condensation product of ethylene oxide with a partial ester derived from a fatty acid and a hexitol anhydride, for example polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be preserved by the addition of an antioxidant such as ascorbic acid.

Dispersible powders and granules are suitable for the preparation of an aqueous suspension. They provide the active ingredient in admixture with a dispersing or wetting agent, a suspending agent and

one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be a vegetable oil such as olive oil or arachis oils, or a mineral oil such as liquid paraffin or a mixture thereof. Suitable emulsifying agents may be (1) naturally-occurring gums such as gum acacia and gum tragacanth, (2) naturally-occurring phosphatides such as soy bean and lecithin, (3)esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also be a sterile injectable solution or suspension in a non-toxic parenterally-acceptable diluent or solvent, for

example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

A composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compositions of the invention are employed.

Treatment dosage for human beings can be varied as necessary. Generally, daily dosages of the compounds of the invention can range from about 0.5 mg to about 1,000 mg; preferably, from about 5 mgs to about 500 mg.

The amount of active ingredient that may be combined with the carrier materials to produce a single dosage form will vary depending upon the host treated and the particular mode of administration. For example, a formulation intended for oral administration may contain, for example, from 5 mg to 500 mg of active agent compounded with an appropriate

and convenient amount of carrier material which may vary from about 5 to about 95 percent of the total composition.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of this invention can also be administered in combination with antihypertensives and/or diuretics and/or calcium entry blockers.

The compounds of Formula 1 can be prepared by the methods shown in the following Reaction Scheme wherein $R$-$R^6$, y and p are as defined above unless otherwise indicated.

As will be evident to those skilled in the art and as demonstrated in the Examples hereinafter, reactive groups not involved in the reactions, such as amino, carboxy, mercapto, etc., may be protected by methods standard in peptide chemistry prior to the coupling reactions and subsequently deprotected to obtain the desired products.

REACTION SCHEME

Process A

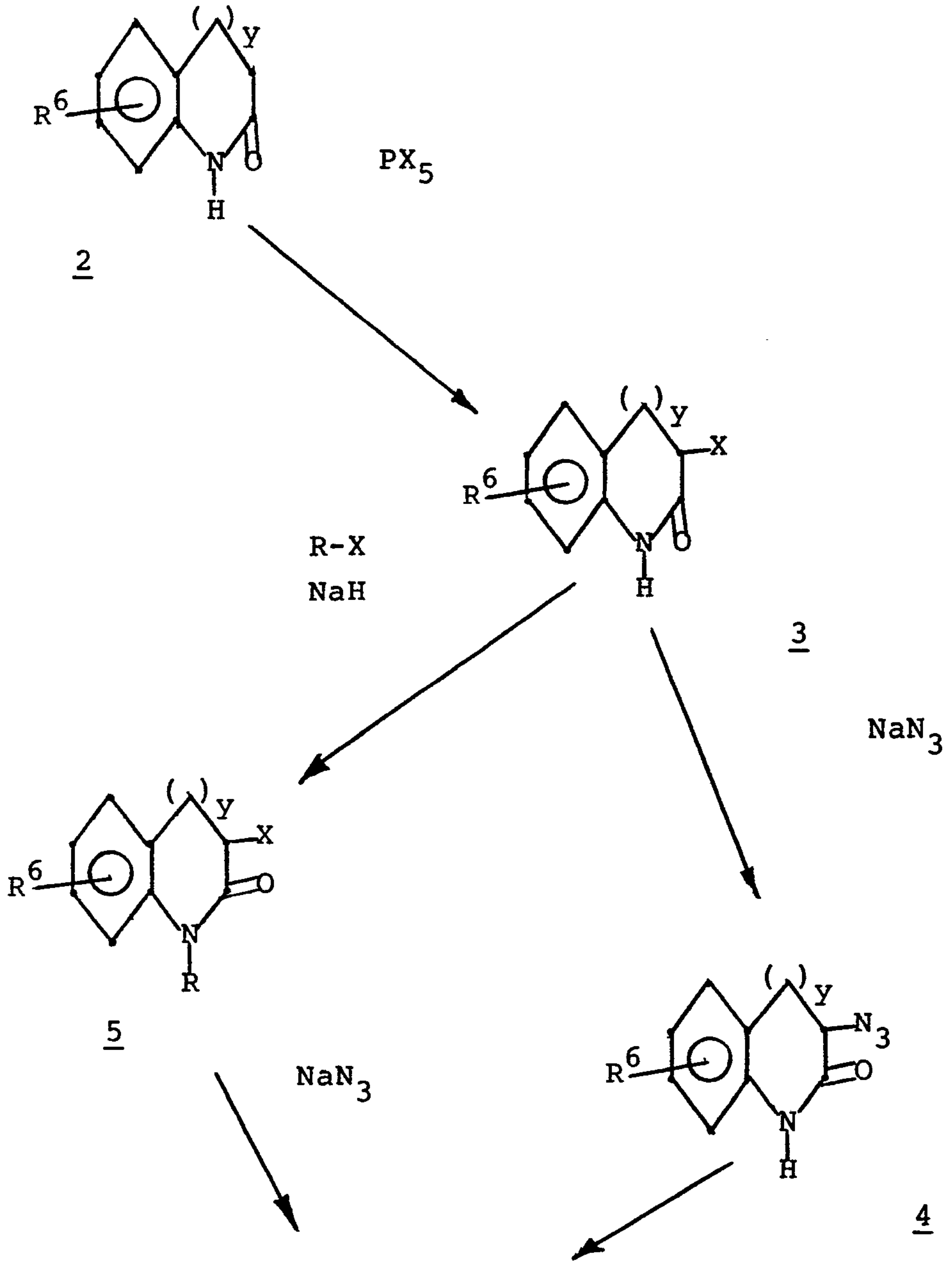

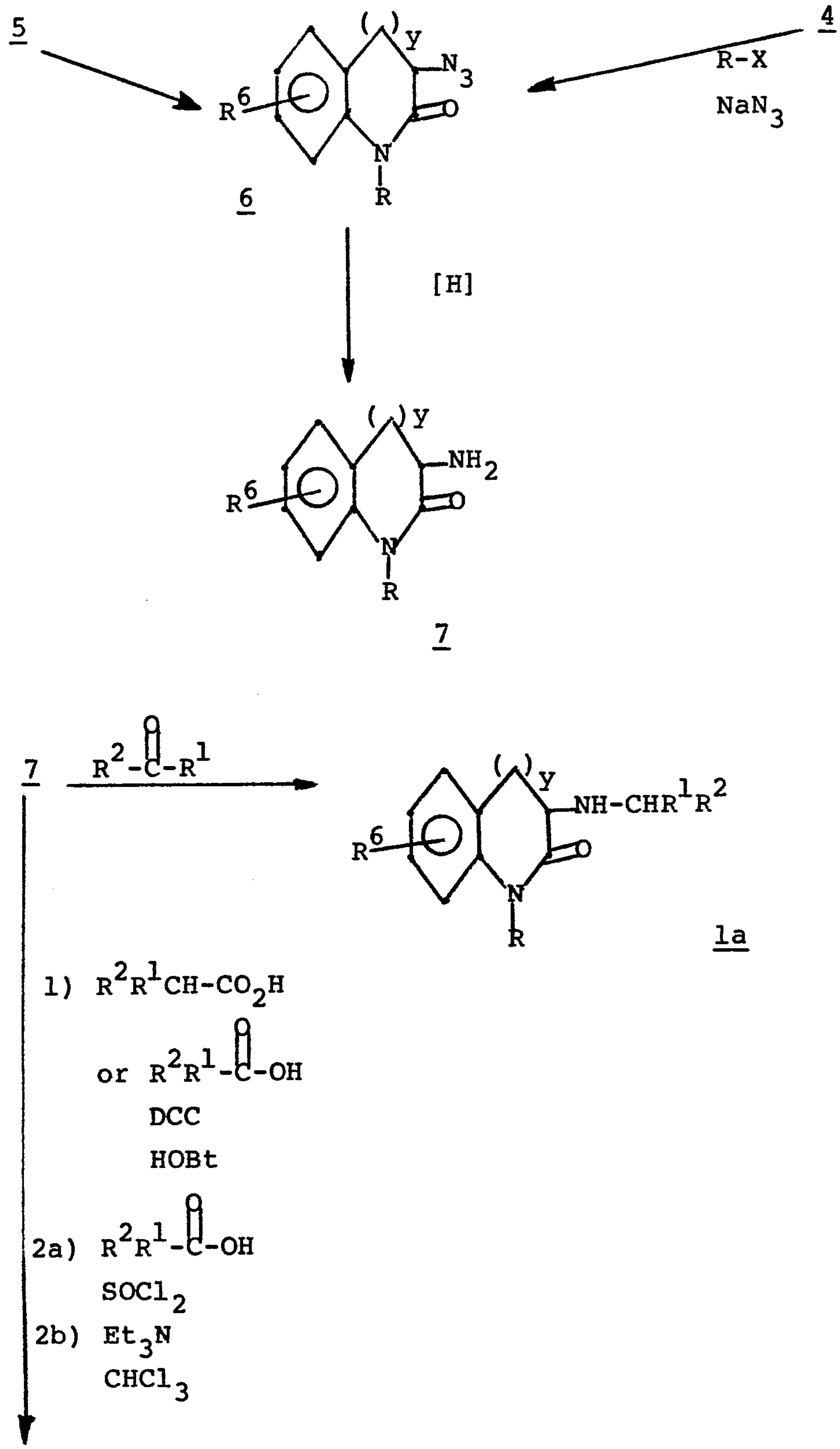
R-X
$NaN_3$
[H]
1) $R^2R^1CH-CO_2H$
or $R^2R^1-C(O)-OH$
DCC
HOBt
2a) $R^2R^1-C(O)-OH$
$SOCl_2$
2b) $Et_3N$
$CHCl_3$

5

4

6

7

7

1a

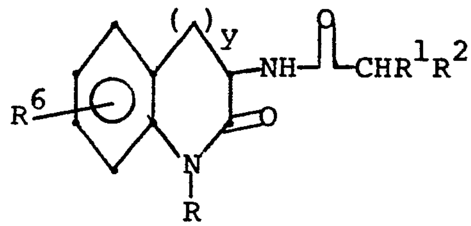

1b

Process B

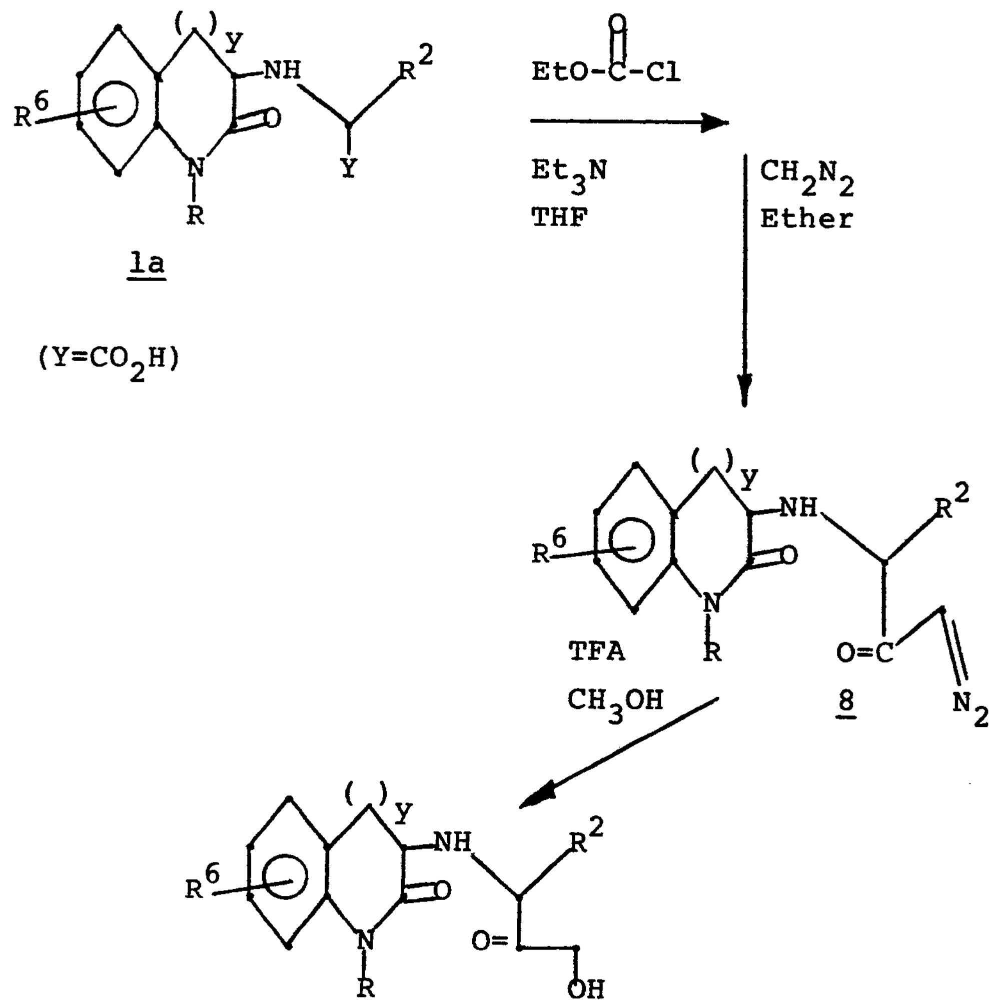


1a

($Y=CO_2H$)

8

1c

Process A

Benzofused lactam 2 ring size ranging from 6 to 8, prepared from a precursor ketone by a procedure of Blicke *et al.*, J. Am. Chem. Soc., 76, 2317 (1954), is converted to (3), with $PX_5$ where X = Br or Cl [Nagasawa *et al.*, J. Med. Chem., 14, 501 (1971)]. Reaction of (3) with sodium or lithium azide in a suitable solvent such as DMF or ethanol [see, for example, Brenner *et al.*, Helv. Chem. Acta, 41, 181 (1958)] affords (4) which can be alkylated with an alkylhalide or iodoester in the presence of a strong base, like sodium hydride, in a solvent such as DMF or THF to produce (5). Reduction of (5) with hydrogen and a suitable catalyst, such as palladium on carbon, affords (6).

Alternatively, (3) may be alkylated in the presence of a strong base, like sodium hydride, and the intermediate (6) converted to (7) by reaction with an azide salt as described above.

Intermediate 7 may be reductively coupled with an aldehyde, ketone, keto acid or ketoester in a solvent such as ethanol using a catalyst such as palladium on carbon to afford 1a (X=absent). 1a may also be prepared using sodium cyanoborohydride to effect reduction.

Alternatively, intermediate 7 may be coupled with a carboxylic acid using a coupling agent such as dicyclohexylcarbodiimide (DCC) and an activator such as 1-hydroxybenzotriazole (HOBt) in an aprotic solvent such as chloroform or THF to afford

1b $(X-\overset{O}{\overset{\|}{C}}-)$.

Compound 1b can also be prepared by reacting 7 with an acid chloride and a tertiary amine such as triethylamine in an aprotic solvent such as chloroform or THF. The acid chloride is prepared from a carboxylic acid and thionyl chloride or $PCl_3$ in an aprotic solvent such as chloroform.

Process B

Compound 1a ($Y\text{-}CO_2H$) may be further elaborated as follows. The acid 1a can be reacted with an alkoxychloroformate (such as ethylchloroformate), a tertiary amine (such as triethylamine) in an aprotic solvent such as tetrahydrofuran (THF) to give, upon filtration, a mixed anhydride intermediate which is then reacted with an excess of diazomethane in ether to give diazomethylketone (8). Reaction of (8) with trifluoroacetic acid (TFA). Followed by stirring in methanol or ethanol gives hydroxymethyl ketone 1c.
($R_1 = \underset{\underset{O}{\|}}{C}\text{-}CH_2\text{-}OH$).

In the above preparations, the keto acid or ester can be represented by the formula $R\text{-}\overset{\overset{O}{\|}}{C}\text{-}\overset{\overset{O}{\|}}{C}\text{-}R^1$ and may be, for example, 2-oxo-4-phenyl-butyric acid. Other α-keto acids or esters may be utilized to prepare other compounds of the present invention for various definitions of R and $R^1$. Such α-keto acids are readily available or may be prepared by well-known techniques. For example, synthons such as

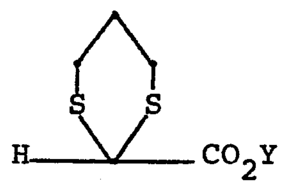

can be converted to α-keto acids or esters using methods involving alkylation followed by hydrolysis as described in the literature. An excellent method involves the reaction of Grignard reagents $R_1MgX$ with $ClCOCO_2Y$ or $YO_2CCO_2Y$. Another method involves condensing substituted acetic acid esters with diethyl oxalate followed by hydrolytic decarboxylation under acidic conditions to obtain α-keto acids. Carefully controlled acid hydrolysis in alcohol of acyl cyanides, which are prepared from acid chlorides and cuprous cyanide, also proves to be a viable synthetic route to α-keto esters. Nucleophilic displacement reactions on chloro or bromo pyruvic acid (ester) can also be used to produce a variety of interesting α-keto acids (esters). In these formulae, Y is a group such as loweralkyl or benzyl and protecting groups are employed as necessary in the $R_1$ group if an interfering functionality is present.

Additional compounds of Formula 1 can be prepared by employing the keto acids and esters listed in Table I below.

TABLE I

Keto Acids and Esters of the Formula R-C=O COOR[1]

(a)

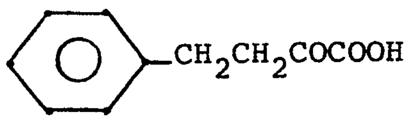

(b)

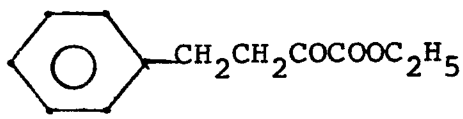

(c)

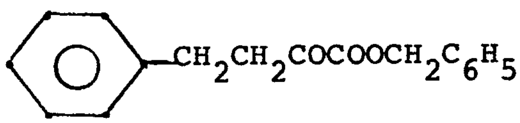

(d)

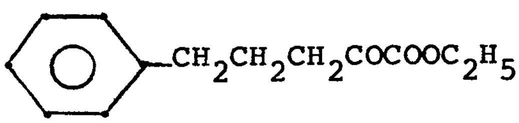

(e)

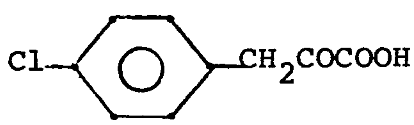

(f)

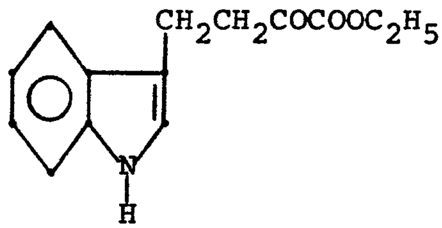

(g)

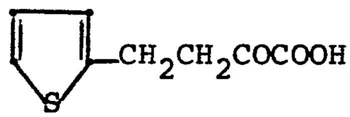

(h)

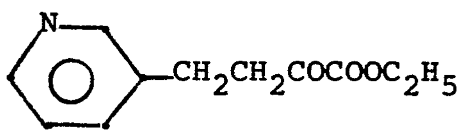

(i)

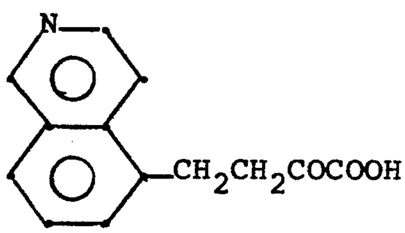

(j)

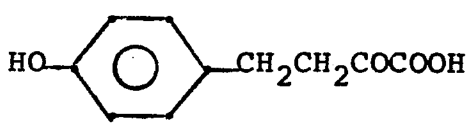

(k)

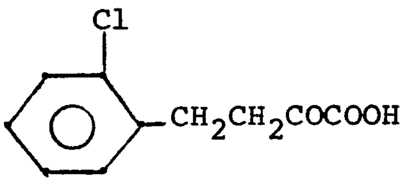

(l)

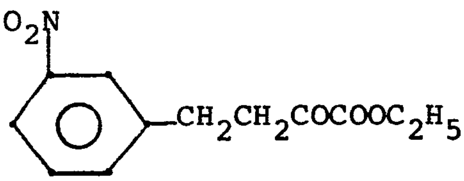

(precursor for the corresponding amino compound)

(m)

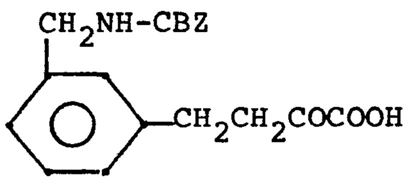

(precursor for the corresponding amino compound)

(n)

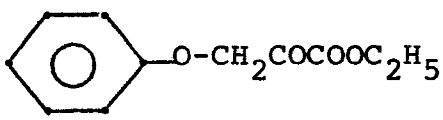

(o)

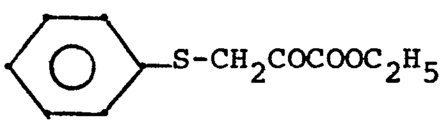

(p)

$CH_3S-CH_2CH_2COCOOH$

(q)

$(CH_3)_2-CH_2CH_2COCOOH$

(r)

$CBZ-HN(CH_2)_4-COCOOH$
(precursor for the corresponding amino compound)

(s)

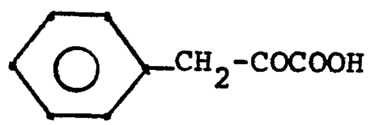

(t)

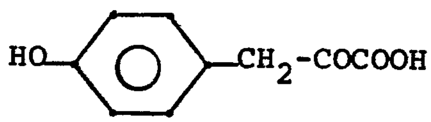

(u)

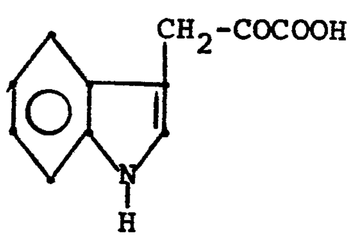

The following examples set forth the best mode currently known for preparing the compounds of the invention and are not intended to be construed as limitative, but illustrative, thereof. Unless otherwise indicated, all temperatures are in degrees Celcius.

EXAMPLE 1

A. 3-Bromo-homodihydrocarbostyril

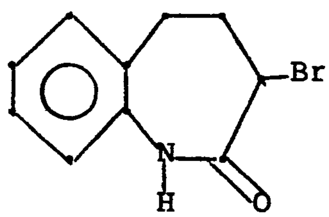

D

To a solution of 15 gm (0.093 mol) of homo dihydrocarbostyril (L. H. Briggs, J.C.S., 456 (1937)) in 200 ml of chloroform was added in increments over a period of an hour 19 gm of $PCl_5$ at which time was added 140 mg of iodine followed by a slow dropwise addition of 90 ml of a 1M solution of bromine in chloroform. The reaction mixture was warmed to room temperature where stirring was continued a further 1 hour.

The crude reaction mixture was concentrated *in vacuo* and then partitioned between water, ice and chloroform. The aqueous layer was extracted 2 times with methylene chloride and the combined organic fractions were filtered through $MgSO_4$ and concentrated *in vacuo*. The crude bromide D was chromatographed (silica, 2:1 ether:hexanes) to give 6.5 gm of pure D bromide.

TLC (silica, 2:1 ether:hexanes) $R_f$=0.65
NMR ($CDCl_3$, TMS) 2.4-3.0 (m, 4H); 4.4-4.7 t, 1H); 7.2 (s, 4H); 9.2 (bs, 1H)
IR 1650 $cm^{-1}$
mass spectrum: $M^+$ 239, m/e: 241 ($M^+$, +2); 160 ($M^{+2}$ -Br); 132 (-C=O)

B. 3-Azido homodihydrocarbostyril

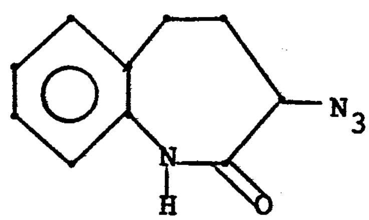

E

To a solution of 9.98 gm (0.0417 mol) of 3-bromo homodihydrocarbostyril in 200 ml of DMF was added 10.8 gm of sodium azide stirring 12 hours at 60°C at which time the DMF was removed at reduced pressure. To the crude reaction mixture was added 50 ml of water and the mixture was then extracted 3 times with 50 ml of chloroform. The combined organic fractions were combined, washed with 25 ml of a saturated solution of NaCl, filtered through $MgSO_4$ and concentrated at reduced pressure. Chromatography (silica, 2:1 ether:hexanes) gave 7.92 gm of pure E azide. m.p. 150-151°C
TLC (silica, 2:1 ether:hexanes) $R_f$=0.71
El. An. Calc. for $C_{10}H_{10}N_4O$
N, 27.71; C, 59.39; H, 4.98
Found N, 27.27; C, 59.25; H, 4.98

NMR ($CDCl_3$, TMS) 2.2-2.8 (m, 4H), 3.6-4.0 (dd, 1H); 7.2 (bs, 4H) 9.2 (bs, 1H)IR $N_3$ 2130, CO 1678
mass spectrum: $M^+$ 202, m/e 174 ($M^+$-$N_2$; 146 (C=O)

C. 3-Azido-1-methylhomodihydrocarbostyril

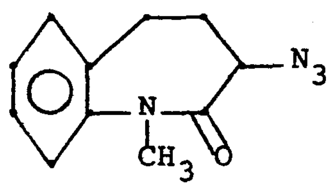

To a suspension of 151 mg of sodium hydride (50% dispersion in oil washed 3 times with hexanes) in 3 ml of THF at 0°C was added dropwise a solution of 0.6 gm of 3 azidohomodihydrocarbostyril and 0.37 ml of methyl iodide in 3 ml of THF. The reaction was carefully monitored by tlc (silica, 7:3 hexane:ethyl acetate) until reaction was complete at which time it was quenched with 10 ml of saturated $NH_4Cl$, diluted with 10 ml of $H_2O$ and extracted 2 times with 10 ml of ethyl acetate. The combined organic layers were filtered through $MgSO_4$, concentrated *in vacuo* and chromatographed (silica, 7:3, hexane:ethyl acetate) to give 0.45 gm of product.
NMR ($CDCl_3$, TMS) 2.0-3.1 (m, 4H); 3.4 (S, 3H); 3.5-3.8 (overlapping d, 1H); 7.2 (S, 4H).

D. 3-Amino-1-methylhomodihydrocarbostyril

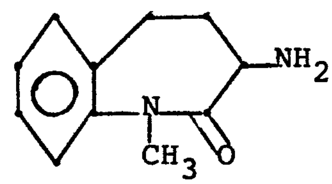

A solution of 2.7 gm of 3-azido-1-methyl homodihydrocarbostyril in 18 ml of absolute ethanol with 0.27 gm of pd/C 10% was hydrogenated for 12 hours at room temperature and 40 lbs of $H_2$. The reaction was subsequently filtered and the ethanol removed at reduced pressure to give 2.6 gm of amine.
NMR ($CD_3OD$, TMS) 1.8-3.0 (m, 4H); 3.4 (S, 3H); 3.4-3.8 (M, 1H); 7.2 (M, 4H).

E. 1-Methyl-3-(1-Carboethoxy-3-phenyl-1-propyl) aminohomodihydrocarbostyril

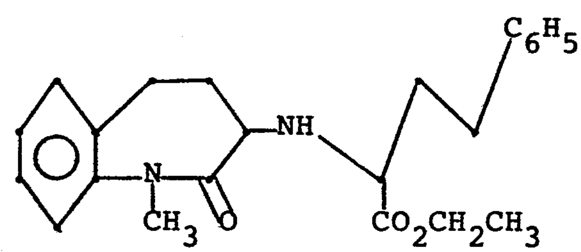

A solution of 2.6 gm of 1-methyl-3-amino homodihydrocarbostyril 8.65 gm of ethyl-4-phenyl-2-oxobutyrate and 0.72 gm of acetic acid in 15 ml of absolute ethanol was stirred 1 hour at room temperature. To the stirred solution was added dropwise over a 16 hour period a solution of 1.9 gm of $NaCNBH_3$ in 12 ml of ethanol. After stirring a further 8 hours, the reaction was concentrated and partitioned between $H_2O$ and ethylacetate. After

extracting twice with ethyl acetate the combined organic layers were filtered through $MgSO_4$, concentrated in vacuo and chromatographed (silica, 7:3, hexane:ethylacetate) to give two diasteriomeric racemates.

Racemate A 440 mg

TLC (silica, 7:3, hexane:ethylacetate) $R_f = 0.21$

NMR ($CDCl_3$, TMS) 1.2 (t, 3H; 1.6-3.2 (m, 11H); 3.4 (s, 3H); 4.15 (q, 2H); 7.1-7.3 (overlapping singlets; 9H).

El. An. Calc. for $C_{23}H_{28}O_3N_2$ 1/4 $H_2O$:

N, 7.28; C, 71.75; H, 7.33

Found N, 7.22; C, 72.06; H, 7.44

Racemate B 520 mg

TLC (silica, 7:3, hexane:ethylacetate) $R_f = 0.15$

NMR ($CDCl_3$, TMS) 1.1 (t, 3H); 1.6-3.5 (m, 11H); 3.3 (S, 3H); 4.0 (q, 2H); 7.15 (S, 9H).

El. An. Calc. for $C_{23}H_{28}O_3N_2$ 1/2 $H_2O$:

N, 7.19; C, 70.93; H, 7.25

Found: N, 7.15; C, 71.33; H, 7.35

## EXAMPLE 2

### A. 1-t-Butoxycarbonylmethyl-3-azido homodihydrocarbostyril

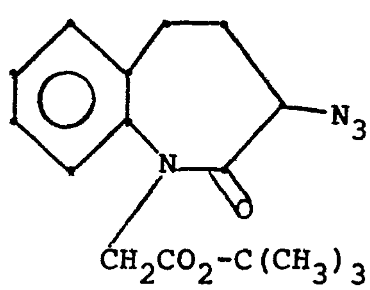

F

To a suspension of 1 gm of sodium hydride (50% dispersion in oil washed 3 times with hexanes) in 20 ml of THF at 0°C was added dropwise a solution of 4.2 gm (0.02 mol) of 3-azido homodihydrocarbostyril (Compound B, Example 1) and 3 ml of t-butyliodo acetate in 20 ml of THF. The reaction was carefully monitored by TLC (silica, 2:1 ether:hexanes) until reaction was complete at which time the reaction was quenched with 30 ml of saturated $NH_4Cl$, diluted with 20 ml $H_2O$ and extracted 3 times with 50 ml $CH_2Cl_2$. The combined organic layers were filtered, concentrated in vacuo and chromatographed (silica, 2:1 ether:hexanes) to give 4.5 gm of pure product F.

m.p. 103-104°C

TLC (silica, 2:1 ether:hexanes) $R_f$=0.74

El. An. Calc. for $C_{16}H_{20}N_4O_3$

N, 17.71; C, 60.74; H, 6.37

Found N, 17.39; C, 60.54; H, 6.61

NMR ($CDCl_3$, TMS) 1.5 (s, 9H), 2.2-3.4 (m, 4H); 3.5-4.0 (overlapping doublets, 1H); 4.2-4.8 (ABq, 2H); 7.2 (s, 4H)

mass spectrum: $M^+$ 316, m/e 288 ($M^+$-$N_2$; 260 ($M^+$-$C_4H_9$)

B. 1-t-Butoxycarbonylmethyl-3-aminohomodihydrocarbostyril

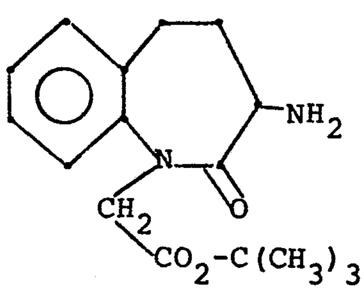

G

A solution of 8.01 gm of 1-t-butoxycarbonylmethyl-3-azidohomodihydrocarbostyril in 150 ml of absolute ethanol with 0.8 gm of Pd/C 10% was hydrogenated for 12 hr at room temperature and 40 lbs of $H_2$. The reaction was subsequently filtered and the ethanol removed at reduced pressure to give 7.05 gm of pure amine G.

m.p. 107-109°C.

El. An. Calc. for $C_{16}H_{22}N_2O_3.1/2H_2O$

N, 9.36; C, 64.19; H, 7.41

Found N, 9.18; C, 64.17; H, 7.53

NMR ($D_2O$, $CDCl_3$, TMS) 1.4 (s, 9H), 2.2-3.8 (m, 5H); 4.1-4.7 (ABq, 2H); 7.05 (bs, 4H)

IR C=O 1735, 1660

mass spectrum: $M^+$ 290, m/e 262 ($M^+$-C=O); 234 ($M^+-C_4H_8$); 217 ($M^+-C_4H_9O$).

C. 1-Carbomethoxymethyl-3-aminohomodihydro carbostyril

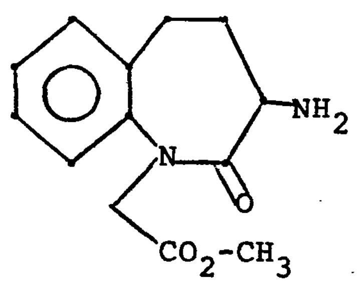

A solution of 1 gm of 1-t-butoxycarbonylmethyl-3-aminohomodihydrocarbostyril in 20 ml of methanol was saturated with HCl, the flask was sealed and the reaction allowed to stir 5 hours at room temperature. The reaction mixture was concentrated *in vacuo* and partitioned between dilute $NaHCO_3$ and

ethylacetate. The $H_2O$ layer was extracted 2 times with ethylacetate and the combined organic layers were filtered through $MgSO_4$ and concentrated at reduced pressure to give 850 mg of product.
NMR ($CDCl_3$, TMS) 1.8-3.6 (m, 7H); 3.65 (S, 2H), 4.5 (ABq, 2H); 7.1 (S, 4H).

D. 1-Carbomethoxymethyl-3-(3-indole methyl)amino homodihydrocarboxtyril

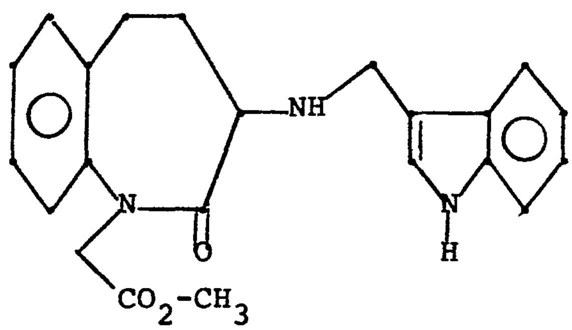

A solution of 0.5 gm of 1-carbomethoxymethyl-3-aminohomodihydrocarbostyril, 0.87 gm of Indole-3-carboxaldehyde and 0.12 ml of acetic acid in 6 ml of methanol was stirred 1 hour at room temperature. To the stirred solution was added dropwise over 6 hours a solution of 0.32 gm of $NaCNBH_3$ in 6 ml of methanol. After stirring an additional 8 hours, the reaction was concentrated *in vacuo*, and partitioned between $H_2O$ and ethylacetate. The organic layer was filtered through $MgSO_4$, concentrated and chromatographed (silica, 9:1:1, ethylacetate:acetonitrile:methanol) to give 0.16 gm of said product.
TLC (silica, 9:1:1, ethylacetate:acetonitrile: methanol) $R_f$ = 0.36.

NMR ($CDCl_3$, TMS) 1.8-3.6 (m, 6H); 3.7 (s, 3H); 3.8 (ABq, 2H); 4.6 (s, 2H); 6.9 (m, 1H); 7.1 (bs, 7H); 7.5-7:7 (m, 1H); 8.4 (bs, 1H).

EXAMPLE 3

A. 1-Carbomethoxymethyl-3-aminohomodihydrocarbostyril

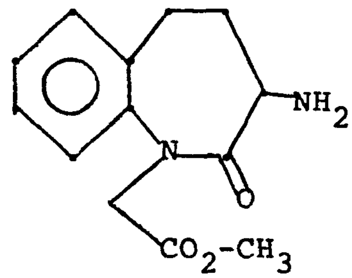

A solution of 1 gm of 1-t-butoxycarbonylmethyl-3-aminohomodihydrocarbostyril (Compound B, Example 2) in 20 ml of methanol was saturated with HCl, the flask sealed and the reaction allowed to stir 6 hours at room temperature. The reaction mixture was concentrated *in vacuo* and partitioned between dilute $NaHCO_3$ and ethylacetate. The $H_2O$ layer was extracted 2 times with ethylacetate and the combined organic layers were filtered through $MgSO_4$ and concentrated at reduced pressure to give 850 mg of product.

NMR ($CDCl_3$, TMS) 1.8-3.6 (m, 7H); 3.65 (s, 2H), 4.5 (ABq, 2H); 7.1 (s, 4H).

B. 1-Carbomethoxymethyl-3-(1-t-butoxycarbonyl;-3-phenyl-1-propyl)-aminohomodihydrocarbostyril (Racemate mixture)

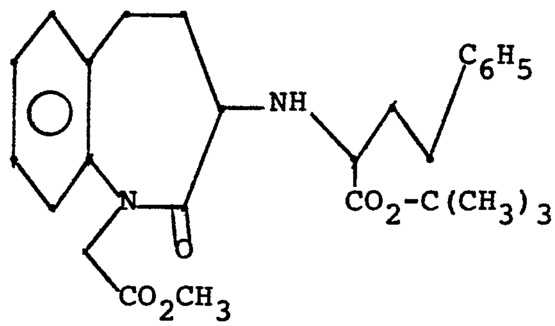

A solution of 4.3 gm of 1-carbomethoxymethyl-3-aminohomodihydrocarbostyril, 8 gm of t-butyl-4-phenyl-2-oxobutyrate and 1 gm of acetic acid in 35 ml of ethanol was stirred 1 hour at room temperature. To the stirred solution was added dropwise over a 10 hour period a solution of 2.67 gm of $NaCNBH_3$ in 35 ml of ethanol. After stirring for an additional 9 hours, the reaction was concentrated in vacuo and partitioned between $H_2O$ and ethylacetate. After extracting twice with ethylacetate the combined organic layers were filtered through $MgSO_4$, concentrated and chromatographed (silica, 1:1 ethylacetate:hexanes) to give two diasteriomeric racemates of which racemate B was retained.

Racemate B 3 gm

TLC (silica, 1:1 ethylacetate:hexanes) $R_f$ = 0.64

NMR ($CDCl_3$, TMS) 1.2 (s, 9H); 1.5-3.2 (m, 11H); 3.5 (s, 3H); 3.4 (ABq, 2H); 7.0 (s, 9H)

El. Ann Calc. for $C_{27}H_{34}N_2O_5$: N, 6.00; C, 69.50; H, 7.35

Found: N, 5.84; C, 69.52; H, 7.27.

C. 1-Carbomethoxymethyl-3-(1-carboxy-3-phenyl-1-propyl)-aminohomodihydrocarbostyril

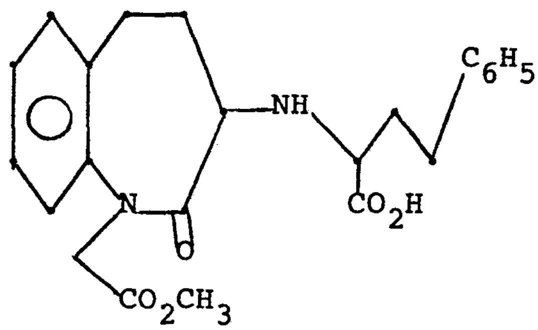

A solution of 2.5 gm of 1-carbomethoxymethyl-3-(1-t-butoxycarbonyl-3-phenyl-1-propyl)-aminohomodihydrocarbostyril, racemate B, in 20 ml of trifluoroacetic acid was stirred 12 hours at room temperature where upon it was concentrated *in vacuo* to yield 1.69 gm of the title compound. NMR ($CD_3OD$, tms) 2.0-3.2 (m, 8H); 3.6 (s, 3H); 3.6-4.1 (m, 2H); 3.6 (s, 2H); 7.1-7.4 (overlapping s, 9H).

D. 1-Carbomethoxymethyl-3-(1-azidomethylcarbonyl-3-phenyl-1-propyl)-aminohomodihydrocarostyril

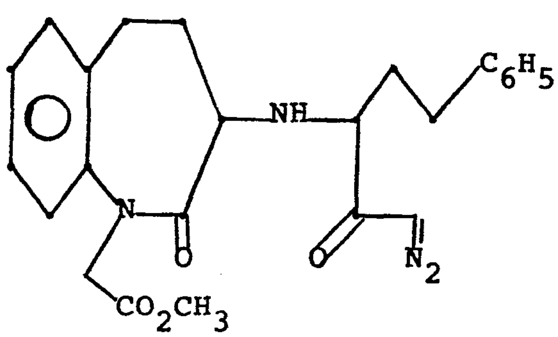

To a solution of 1.54 gm of 1-carbomethoxymethyl-3-(1-carboxy-3-phenyl-1-propyl)aminohomodihydrocarbostyril in 15 ml of THF at 0°C was added with stirring 0.56 ml of triethylamine followed by 0.32 ml of methylchloroformate. Stirring at 0°C was continued for 15 minutes whereupon the reaction mixture was filtered in a nitrogen atmosphere to remove precipitated triethylaminehydrochloride. To the filtrate at 0°C was added a solution of diazomethane in ether. The reaction's progress was monitored by tlc (silica, 1:1 hexane:ethylacetate) and diazomethane solution was added as needed. When the reaction was complete (10 hours), the solution was filtered and solvents removed at reduced pressure. The crude product was purified by chromatography (silica, 1:1, hexane: ethylacetate) to give 1.25 gm of diazoketone.

TLC (silica, 1:1, hexane:ethylacetate) $R_f$ = 0.50

NMR ($CDCl_3$, TMS) 1.7-3.1 (m, 8H); 3.6 (s, 3H); 3.9-4.1 (m, 2H) 4.5 (ABq, 2H); 5.5 (s, 1H); 7.1-7.3 (overlapping singlets, 9H).

E. 1-Carbomethoxymethyl-3-[1-(2-hydroxy-1-oxoethyl)-3-phenyl-1-propyl]aminohomodihydrocarbostyril

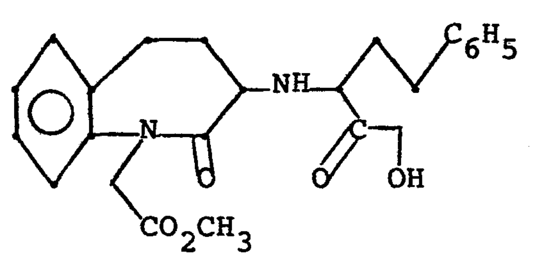

A solution of 0.3 gm of 1-carbomethoxymethyl-3-(1-(azidomethylcarbonyl)-3-phenyl-1-propyl)aminohomo-

dihydrocarbostyril in 5 ml of trifluoroacetic acid was stirred at room temperature for 3 hours at which time the acid was removed at reduced pressure. The crude reaction mixture was dissolved in 5 ml of methanol an the solution stirred 6 hours at room temperature, where upon the methanol was removed *in vacuo*. The crude product was purified by chromatography (silica, 1:1, ethylacetate:hexane) to give the title compound.

TLC (silica, 1:1, ethylacetate:hexane) $R_f = 0.44$

NMR ($CDCl_4$, TMS) 1.7-3.2 (m, 11H); 3.6 (s, 3H); 4.0-5.0 (m, 5H); 7.2 (bs, 9H).

mass spectrum: $M^+$ 424, m/e: 423 ($M^+$ -1), 365 ($M^+-CO_2CH_3$).

## EXAMPLE 4

1-t-Butoxycarbonylmethyl-3-(D-N-acetyltryptophanyl)-aminohomodihydrocarbostyril

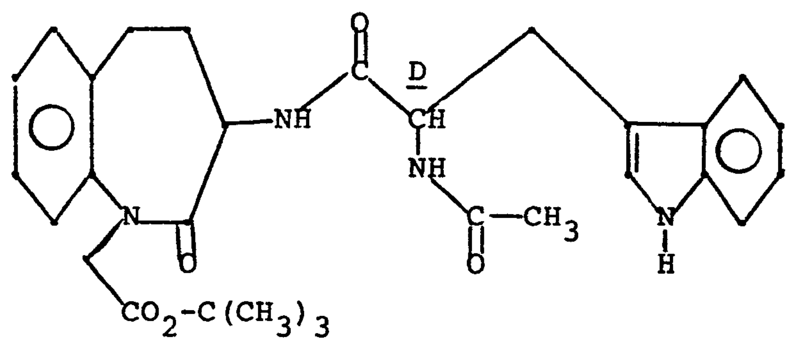

To a solution of 0.3 gm of 1-t-butoxycarbonylmethyl-3-aminohomodihydrocarbostyril (Compound B, Example 2) and 0.25 gm of N-acetyl-D-tryptophan in 5 ml of chloroform at room temperature was successively added 0.15 gm of 1-hydroxybenzotriazole and 0.24 gm of dicyclohexylcarbodiimide. After stirring for 12 hours at room temperature the reaction mixture was filtered and the filtrate was washed with $H_2O$, filtered through $MgSO_4$ and concentrated in vacuo. The product was chromatographed to give two diasteriomeric racemates.

Racemate A 150 mg
TLC (silica, 9:1 ethylacetate:acetonitrile) $R_f$ = 0.416
NMR ($CDCl_3$, TMS) 1.4 (s, 9H); 1.9 (s, 3H); 2.3-3.3 (m, 6H); 3.9-4.7 (m, 2H); 4.3 (ABq, 2H); 6.4-6.6 (overlapping d, 2H); 6.9-7.5 (m, 9H); 8.6 (bs, 1H).

Racemate B 200 mg
TLC (silica, 9:1, ethylacetate:acetonitrile) $R_f$ = 0.416
NMR ($CDCl_3$, TMS) 1.35 (s, 9H), 1.8 (s, 3H); 2.1-3.3 (m, 6H); 4.3 (ABq, 2H); 4.0-4.8 (m, 2H); 6.3-6.7 (overlapping d, 2H); 6.8-7.5 (m, 9H); 8.5 (bs, 1H).

## EXAMPLE 5

1-t-Butoxycarbonylmethyl-3-(L-N-acetyltryptophanyl)-aminohomodihydrocarbostyril

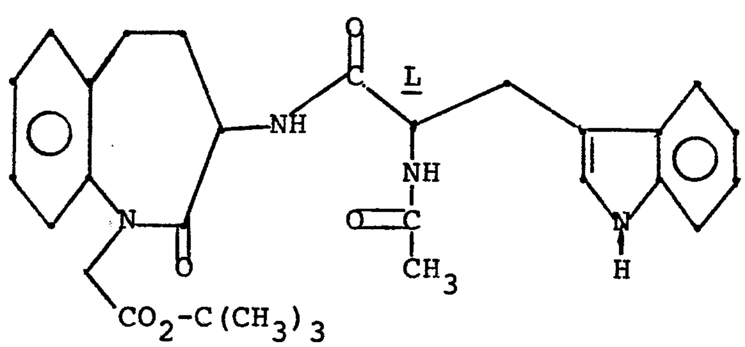

To a solution of 0.3 gm of 1-t-butoxycarbonylmethyl-3-aminohomodihydrocarbostyril (Compound B, Example 2) and 0.25 gm of N-acetyl-D-tryptophan in 5 ml of chloroform at room temperature was successively added 0.15 gm of 1-hydroxybenzotriazole and 0.24 gm of dicyclohexylcarbodiimide. After stirring for 12 hours the reaction mixture was filtered. The filtrate was washed with $H_2O$, filtered through $MgSO_4$ and concentrated in vacuo and chromatographed to give two diasteriomeric racemates.

Racemate A
TLC (silica, 9:1 ethylacetate:acetonitrile) $R_f$ = 0.26
NMR ($CDCl_3$, TMS) 1.4 (s, 9H); 1.8 (s, 3H); 2.3-3.3 (m, 6H); 3.9-3.7 (m, 2H); 4.3 (ABq, 2H); 6.4-6.7 (overlapping d, 2H); 6.8-7.4 (m, 9H); 8.6 (bs, 1H).

Racemate B
TLC (silica, 9:1, ethylacetate:acetonitrile) $R_f$ = 0.26
NMR ($CDCl_3$, TMS) 1.3 (s, 9H), 1.7 (s, 3H); 2.1-3.2 (m, 6H); 4.3 (ABq, 2H); 4.1-4.8 (m, 2H); 6.6-7.7 (m, 11H).

EXAMPLE 6

1-t-Butoxycarbonylmethyl-3(-L-N-carbobenzyloxytryptophanyl)aminohomodihydrocarbostyril

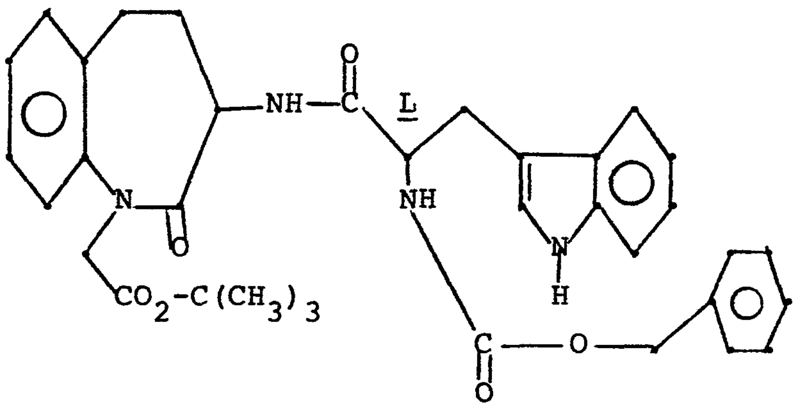

To a solution of 0.3 gm of 1-t-butoxycarbonylmethyl-3-aminohomodihydrocarbostyril (Compound B, Example 2) and 0.36 gm of N-carbobenzyloxy-L-tryptophan in 5 ml of chloroform at room temperature was successively added 0.15 gm of 1-hydroxybenzotriazole and 0.24 gm of dicyclohexylcarbodiimide. After stirring for 4 hours the reaction mixture was filtered, concentrated in vacuo and chromatographed to give two diasteriomeric racemates.

Racemate A 100 mg

TLC (silica, 1:1 ethylacetate:hexane) $R_f$ = 0.27

NMR ($CDCl_3$, TMS) 1.65 (s, 9H); 1.8-3.4 (m, 6H); 4.35 (ABq, 2H); 4.1-4.6 (m, 2H); 5.1 (s, 2H); 5.5 (d, 1H); 6.6 (d, 1H); 6.8-7.6 (m, 14H); 8.3 (s, 1H).

Racemate B 130 mg
TLC (silica, 1:1, ethylacetate:hexane) $R_f$ = 0.27
NMR ($CDCl_3$, TMS) 1.4 (s, 9H), 1.7-3.3 (m, 6H); 4.35 (ABq, 2H); 4.2-4.6 (m, 2H); 5.0 (s, 2H); 5.4 (d, 1H); 6.7 (d, 1H); 6.8-7.4 (m, 14H); 8.4 (bs, 1H).

## EXAMPLE 7

1-t-Butoxycarbonylmethyl-3-((D)-N-carbobenzyloxy-tryptophanyl)aminohomodihydrocarbostyril

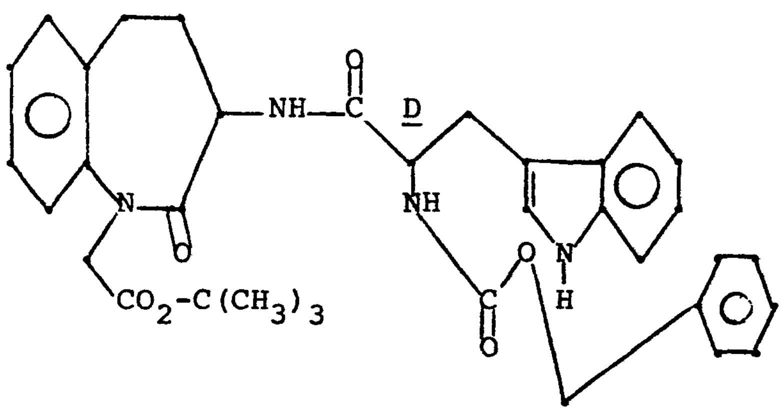

To a solution of 0.3 gm of 1-t-butoxycarbonylmethyl-3-aminohomodihydrocarbostyril (Compound B, Example 2) and 0.36 gm of N-carbobenzyloxy-D-tryptophan in 5 ml of chloroform at room temperature was successively added 0.15 gm of 1-hydroxybenzotriazole and 0.24 gm of dicyclohexylcarbodiimide. After stirring 12 hours at room temperature, the reaction mixture was filtered and partitioned between $H_2O$ and chloroform. The chloroform fraction was filtered through $MgSO_4$, concentrated *in vacuo* and chromatographed to give two diasteriomeric racemates.

Racemate A 150 mg
TLC (silica, 1:1 ethylacetate:hexane) $R_f$ = 0.27
NMR ($CDCl_3$, TMS) 1.4 (s, 9H); 1.8-3.4 (m, 6H); 4.35 (ABq, 2H); 4.1-4.6 (m, 2H); 5.1 (s, 2H); 5.55 (d, 1H); 6.6 (d, 1H); 6.8-7.4 (m, 14H); 8.4 (bs, 1H).

Racemate B 200 mg
TLC (silica, 1:1, ethylacetate:hexane) $R_f$ = 0.27
NMR ($CDCl_3$, TMS) 1.4 (s, 9H), 2.0-3.4 (m, 6H); 4.4 (ABq, 2H); 4.2-4.6 (m, 2H); 5.1 (s, 2H); 5.55 (d, 1H); 6.8 (d, 1H); 6.8-7.5 (m, 14H); 7.6 (bs, 1H).

## EXAMPLE 8

1-Carbo-t-butoxymethyl-3-(carbonyl-2-indolyl)amino homodihydrocarbostyril

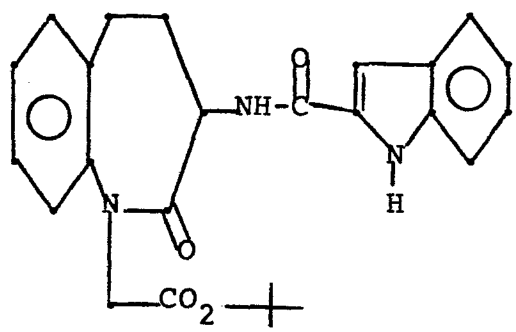

To a solution of indole-2-carboxylic acid 0.165 gm (0.001 mol) in 3 ml of chloroform at room temperature there was added 0.25 ml of thionyl chloride. This solution was stirred 8 hours at room temperature whereupon it was evaporated *in vacuo*. To a solution of the resulting acid chloride in 3 ml of chloroform there was added 1-t-butoxycarbonylmethyl-3-aminohomodihydrocarbostyril (0.3 gm, 0.001 mol) and 0.14 ml of triethylamine at room temperature. The

reaction was monitored by tlc (silica, 1/1, EtOAc/hexanes). After 3 hours, the starting material had been converted to a nonpolar product. The reaction mixture was then partitioned between ethylacetate and water. After extracting the water layer with ethylacetate, the combined organic layers were dried over $Na_2SO_4$, filtered through $MgSO_4$, evaporated at reduced pressure and chromatographed, (silica, 2/1, hexanes/ethylacetate) to give 0.3 gm of product.

TLC (silica, 1:2, ethylacetate:hexanes) $R_f$=0.77

NMR ($CDCl_3$, TMS) 1.5 (s, 9H0, 1.9-3.5 (m, 5H), 4.5 (ABq, m, 3H), 6.8-7.6 (m, 9H).

mass spectrum: $M^+$ 433, m/e 377 ($M^+$-$C_4H_8$), 360 ($M^+$-$C_4H_9O$)

EXAMPLE 9

1-Carboxymethyl-3-(carbonyl-2-indolyl)aminohomodihydrocarbostyril

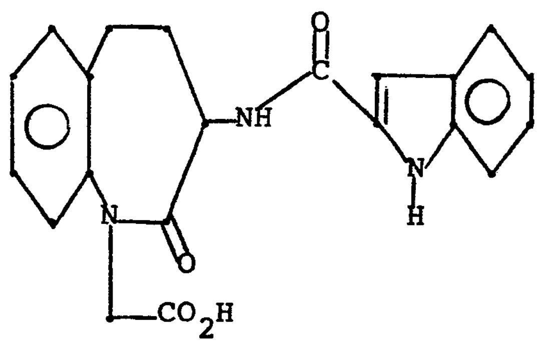

To a solution of 1-carbo-t-butoxymethyl-3-(carbonyl-2-indolyl)aminohomodihydrocarbostyril (0.2 gm) in 2 ml of chloroform at room temperature was added 2 ml of trifluoroacetic acid. The reaction was stirred 3 hours whereupon it was evaporated *in vacuo* to give 0.13 gm of product.

mass spectrum: $M^+$ 377

EXAMPLE 10

1-Carbo-t-butoxymethyl-3-(1-p-chlorobenzoyl)amino-homodihydrocarbostyril

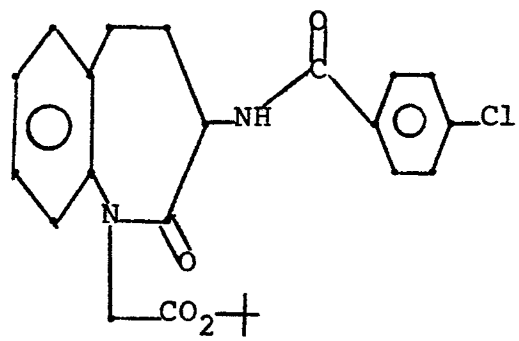

To a solution of 1-t-butoxycarbonylmethyl-3-aminohomodihydrocarbostyril (0.5 gm, 0.0017 mol) in 12 ml of methylene chloride there was added 0.297 gm of 4-chlorobenzoic acid followed by 0.25 gm of 1-hydroxybenzotriazole and 0.39 gm of dicyclohexylcarbodiimide. The reaction mixture was stirred 12 hours at room temperature whereupon it was filtered, washed with 5% $Na_2CO_3$, 5% citric acid, and saturated $NaHCO_3$. The solution was then filtered through $MgSO_4$, evaporated in vacuo, and chromatographed (silica, 7:3, hexanes:ethylacetate) to give 0.35 gm of product.

TLC (silica, 7:3, hexanes:ethylacetate) $R_f$=0.5

El. An. Calc. for $C_{23}H_{25}N_2O_4Cl$

N, 6.53; C, 64.41; H, 5.87.

Found: N, 6.07; C, 64.14; H, 5.86.

NMR ($CDCl_3$, TMS) 1.5 (s, 9H), 2.0-3.5 (m.4H), 4.4 (ABq, 2H), 4.4-4.9 (m, 1H), 7.1-7.8 (m, 8H)

mass spectrum: $M^+$ 429

EXAMPLE 11

1-Carboxymethyl-3-(1-p-chlorobenzoyl)aminohomodihydrocarbostyril

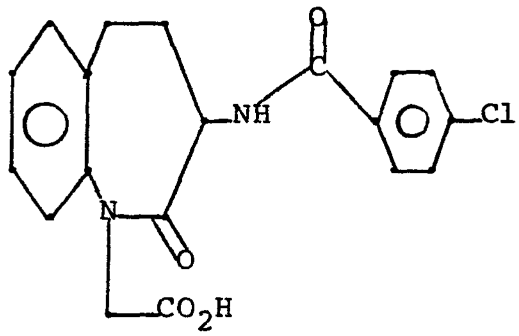

A solution of 1-carbo-t-butoxymethyl-3-(1-p-chlorobenzoyl)-aminohomodihydrocarbostyril in 5 ml of trifluoroacetic acid was stirred for 12 hours at room temperature whereupon it was evaporated in vacuo to give 0.17 gm of the title compound.
NMR ($CDCl_3$, $CD_3OD$, TMS) 2.0-3.6 (m, 4H), 4.5 (ABq, 2H), 4.6-4.9 (m, 1H), 7.1-7.8 (m, 8H).
mass spectrum: $M^+$ 375

EXAMPLE 12

1-Carbo-t-butoxymethyl-3-(benzoyl)aminohomodihydrocarbostyril

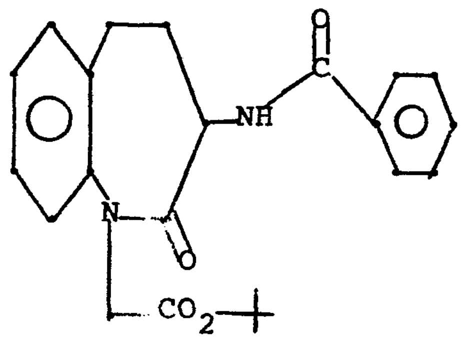

This compound (0.590 gm) was prepared from 0.4 gm of 1-t-butoxycarbonylmethyl-3-amino homodihydrocarbostyril (Example 2, Compound B), 0.185 gm of benzoic acid, 0.205 gm of 1-hydroxybenotriazole and 0.313 gm of dicyclohexylcarbodiimide by a process analogous to Example 10.

TLC (silica, 7:3, hexanes:ethylacetate) $R_f$=0.3

El. An. Calc. for $C_{23}H_{26}N_2O_4$.1/4 $H_2O$

N, 7.02; C, 69.24; H, 6.57

Found: N, 6.63; C, 69.68; H, 6.67

NMR ($CDCl_3$, TMS) 1.5 (s, 9H), 2.0-3.5 (m, 4H), 4.4 (ABq, 2H), 4.5-4.9 (m, 1H), 7.1-8.2 (m, 9H)

mass spectrum $M^+$ 394

EXAMPLE 13

1-Carbo-t-butoxymethyl-3-(3,4-dichlorobenzoyl)amino-homodihydrocarbosytril

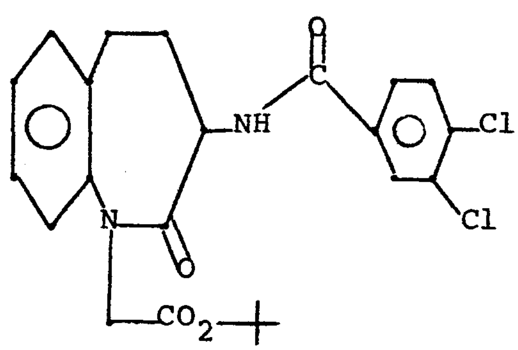

The titled compound (0.27 gm) was prepared from 0.3 gm of 1-t-butoxycarbonyl methyl-3-aminohomodihydrocarbostyril (Example 2, Compound B), 0.217 gm of 3,4-dichlorobenzoic acid, 0.15 gm of 1-hydroxybenzotriazole and 0.23 gm of dicyclohexylcarbodiimide by a process analogous to Example 10.

TLC (silica, 7:3, hexanes:ethylacetate) $R_f$=0.49

NMR ($CDCl_3$, TMS) 1.5 (s, 9H); 1.7-3.5 (m, 4H); 4.4 (ABq, 2H); 4.5-4.9 (m, 1H); 7.1-8.2 (m, 7H)

mass spectrum $M^+$ 462

EXAMPLE 14

1-Carbo-t-butoxymethyl-3-(4-nitrobenzoyl)aminohomo-dihydrocarbostyril

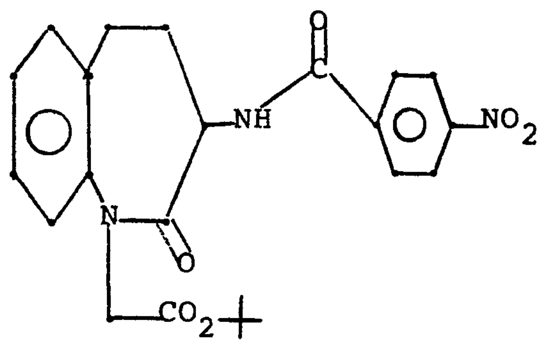

The titled compound (0.29 gm) was prepared from 0.3 gm of 1-t-butoxycarbonylmethyl-3-amino-dihydro carbostyril (Example 2, Compound B), 0.19 gm of 4-nitrobenzoic acid, 0.15 gm of 1-hydroxybenzotriazole and 0.23 gm of dicyclohexylcarbodiimide by a procedure analgous to Example 10.

TLC (silica, 7:3, hexanes:ethylacetate) $R_f$=0.35

NMR ($CDCl_3$, TMS) 1.5 (s, 9H); 1.8-3.4 (m, 4H), 4.5 (ABq, 2H); 4.3-4.8 (m, 1H); 7.2-8.3 (m, 8H).

mass spectrum $M^+$ 410, m/c 384 ($M^+$-$CH_2$= 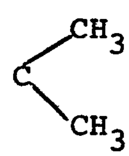 )

EXAMPLE 15

1-t-Butoxycarbonylmethyl-3-(4-fluorobenzoyl)amino-homodihydrocarbostyril

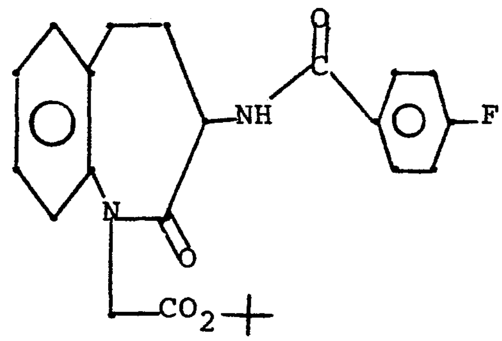

The titled compound (0.38 gm) was prepared from 0.3 gm of 1-t-butoxycarbonylmethyl-3-aminohomodihydrocarbostyril (Example 2, Compound B), 0.16 gm of 4-fluorobenzoic acid, 0.15 gm of 1-hydroxybenzotriazole and 0.23 gm of dicyclohexylcarbodiimide by a procedure analogous to Example 10.

TLC (silica, 7:3, hexanes:ethylacetate) $R_f$=0.43

NMR ($CDCl_3$,TMS) 1.5 (s, 9H); 1.8-3.4 (m, 4H); 4.5 (ABq, 2H); 4.4-4.8 (m, 1H); 6.7-8.2 (m, 8H)

mass spectrum $M^+$ 412

EXAMPLE 16

1-t-Butoxycarbonylmethyl-3-(4-dimethylaminobenzoyl) aminodihydrocarbostyril

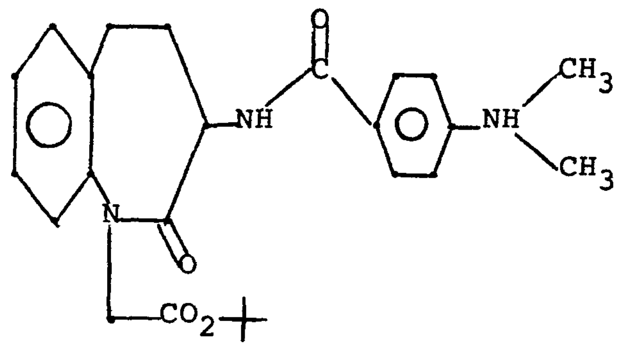

The titled compound (0.2 gm) was prepared from 0.3 gm of 1-t-butoxycarbonylmethyl-3-aminohomodihydrocarbostyril (Example 2, Compound B), 0.19 gm of 4-dimethylaminobenzoic acid, 0.15 gm of 1-hydroxybenzotriazole and 0.23 gm of dicyclohexylcarbodiimide by a procedure analogous to Example 10.

TLC (silica, 1:1, hexanes:ethylacetate) $R_f$=0.50

NMR ($CDCl_3$, TMS) 1.4 (s, 9H); 1.8-3.4 (m, 4H); 2.8 (s, 6H); 4.4 (ABq, 2H); 4.4-4.8 (m, 1H); 6.8-7.4 (m, 8H)

mass spectrum $M^+$ 437

Patent claims

1. A compound of formula I or II

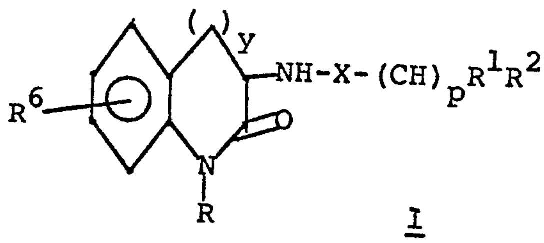

I

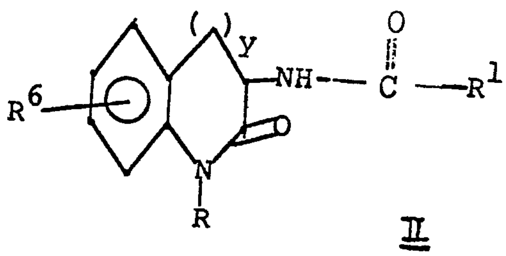

II

wherein in formulae I and II

R is lower $C_1$-$C_8$ alkyl;
$C_6$ or $C_{10}$-aryllower-$C_1$-$C_8$ alkyl;
lower $C_1$-$C_4$-alkyl containing a carbonyl group which carbonyl group can be substituted with $C_1$-$C_4$ alkyl, hydroxy, $C_1$-$C_4$alkoxy, or $NR^3$ wherein $R^3$ is hydrogen, $C_1$-$C_4$alkyl, carboxyloweralkyl, carboxamidoloweralkyl, arylalkyl wherein the aryl is $C_5$-$C_{10}$ and the alkyl is $C_1$-$C_4$;

$R^6$ is hydrogen;
halo;
hydroxy;
$C_1$-$C_8$alkyl;
lower $C_1$-$C_8$alkoxy; and

y is 1-3; and wherein in formula II the radical $R^1$ and in formula I the radicals

$R_1$ and $R_2$ have independently the following meaning:

hydrogen;

lower $C_1$-$C_8$alkyl;

unsubstituted or substituted carbonyl wherein the substituents are alkoxy, alkyl of $C_1$-$C_8$, hydroxyalkyl of $C_1$-$C_8$, amino, loweralkylamino, $C_6$ or $C_{10}$-arylloweralkylamino, carboxylower-alkylamino, carboxamidoloweralkylamino;

groups of formula

$NR^4R^5$

wherein

$R^4$ and $R^5$ can independently be unsubstituted or substituted carbonyl, or unsubstituted or substituted carboxyloweralkyl, or unsubstituted or substituted carboxamidoloweralkyl wherein the substituents are $C_1$-$C_4$alkyl, $C_1$-$C_4$alkyloxy; $C_6$ or $C_{10}$-arylloweralkyl, $C_6$-$C_{10}$-arylloweralkoxy, aminoloweralkyl, substituted aminoloweralkyl;

substituted loweralkyl wherein the substituents are halo, hydroxy, carboxy, carboxamido, carbonyl, substituted carbonyl wherein the substituent is $C_1$-$C_8$ alkyl-OH, loweralkylthio, loweralkoxy, loweralkoxy-carbonyl, lower-$C_6$ or $C_{10}$-arylalkoxycarbonyl, amino, loweralkylamino, lowerdialkylamino, acylamino; substituted loweralkylamino wherein the substituent

can be halo, hydroxy, alkoxy or cyano;
$C_6$ or $C_{10}$-arylloweralkylamino;
$C_6$ or $C_{10}$-aryloxy;
$C_6$ or $C_{10}$-arylthio;
$C_6$ or $C_{10}$-arylalkyloxy;
$C_6$ or $C_{10}$-arylalkylthio;
benzofused cycloalkyl or bicycloalkyl of from 8 - 12 carbon atoms;
aryl or heteroaryl wherein the aryl is $C_6$ or $C_{10}$ and the heteroaryl contains an O, N or S heteroatom and which aryl group or heteroaryl group may be mono, di- or trisubstituted by loweralkyl, hydroxy, loweralkoxy, halo, amino, acylamino, loweralkylthio or aminoloweralkyl;
benzofused cycloalkyl or bicycloalkyl of from 8 to 12 carbon atoms;
$C_6$ or $C_{10}$-arylloweralkyl;
$C_6$ or $C_{10}$-arylloweralkenyl;
heteroarylloweralkyl and heteroarylloweralkenyl in which the aryl rings may be mono-, di- or trisubstituted by halo, loweralkyl, hydroxy, loweralkoxy, amino, loweralkylamino, diloweralkylamino, aminoloweralkyl, acylamino, carboxy, haloloweralkyl, nitro, cyano or sufonamido; arylalkyl or heteroarylalkyl wherein the aryl is $C_6$ or $C_{10}$ and the heteroaryl contains an O, N or S heteroatom and which include branched loweralkyl groups; substituted arylalkyl or substituted heteroarylalkyl which include branched loweralkyl groups wherein the loweralkyl groups can be substituted by amino, acylamino, or hydroxyl and the aryl groups are $C_6$ or $C_{10}$ and the heteroaryl contain

O, N or S heteroatoms and wherein the aryl group and the heteroaryl group is optionally substituted by halo, dihalo, loweralkyl, hydroxy, loweralkoxy, $C_6$ or $C_{10}$-aryloxy, $C_6$ or $C_{10}$-aroyl, $C_6$ or $C_{10}$-arylthio, amino, aminoloweralkyl, loweralkanoylamino, $C_6$ or $C_{10}$-arylamino, lowerdialkylamino, loweralkylamino, hydroxy, hydroxyloweralkyl, trihaloloweralkyl, nitro, cyano, or sulfonamido; and any of the arylalkyl groups, arylalkenyl groups, heteroarylalkyl groups or heteroarylalkenyl groups described above in which the aryl ring or heteroarylring is partially or completely hydrogenated; substituted loweralkyl having the formula $R^1_A(CH_2)_n-O-(CH_2)_m$ wherein n is 0-2, m is 1-3, $R^1_A$ is aryl or heteroaryl wherein the aryl is $C_6$ or $C_{10}$ and the heteroaryl contains an O, N or S heteroatom and the aryl group or heteroaryl group is optionally substituted by amino, lowerdialkylamino, loweralkylamino, hydroxy, hydroxyloweralkyl, aminoloweralkyl, trihaloloweralkyl, cyano, nitro, sulfonamido, $C_6$-$C_{10}$-aroyl, loweralkyl, halo, dihalo, and loweralkoxy and

Q is a bivalent group having the formulae O, S, SO, $SO_2$, $N-R^1_B$, $CONR^1_C$, $NR^1_CCO$ or CH=CH wherein

$R^1_B$ is hydrogen, loweralkyl, $C_6$ or $C_{10}$-aryl, $C_6$ or $C_{10}$-arylalkyl, loweralkanoyl, or $C_6$ or $C_{10}$-aroyl, and

$R^1_C$ is hydrogen, or loweralkyl;

and wherein in formula I p is 1 or 2 and

X is absent or carbonyl and

salts of the compounds of formulae I and II.

2. A compound of claim 1 wherein in formulae I and II $R_1$ is an optionally substituted aryl group having 6 or 10 carbon atoms, an optionally substituted heteroaryl group in which the heteroatom is oxygen, sulfur and preferably nitrogen or an alkyl group substituted with an optionally substituted aryl or an optionally substituted heteroaryl group in which the heteroatoms are oxygen, sulfur and preferably nitrogen, or a salt of the compounds of formulae I and II.

3. A compound of claim 1 or 2 having the formula I wherein p is 1, or a salt of said compounds.

4. A compound of one of the claims 1 - 3 which is a member of the group comprising

1-methyl-3-(1-carboethoxy-3-phenyl-1-propyl)amino homodihydrocarbostyril;

1-carbomethoxymethyl-3-(3-indole methyl)amino homodihydrocarbostyril;

1-carbomethoxymethyl-3-[1-(2-hydroxy-1-oxoethyl)-3-phenyl-1-propyl)aminohomodihydrocarbostyril;

1-t-butoxycarbonylmethyl-3-(D-N-acetyltryptophanyl)aminohomodihydrocarbostyril;

1-t-butoxycarbonylmethyl-3-(L-N-acetyltryptophanyl) aminohomodihydrocarbostyril;

1-t-butoxycarbonylmethyl-3-(L-N-carbobenzyloxy-tryptophanyl)aminohomodihydrocarbostyril;

1-t-butoxycarbonylmethyl-3-(D-N-carbobenzyloxy-tryptophanyl)aminohomodihydrocarbostyril;

1-carbo-5-butoxymethyl-3-(carbonyl-2-indolyl)amino homodihydrocarbostyril;

1-carboxymethyl-3-(carbonyl-2-indolyl)amino homo-dihydrocarbostyril;

1-carbo-t-butoxymethyl-3-(3,4-dichlorobenzoyl)aminohomodihydrocarbostyril,
and salts of said compounds.

5. A pharmaceutical composition for treating gastrointestinal disorders, central nervous system disorders, or regulating appetite in mammals, preferably human beings, characterized in that it contains as pharmaceutically effective ingredient a compound of formulae I or II according to patent claim 1 or a pharmaceutically acceptable salt of the compounds of formulae I or II.

6. A pharmaceutical composition according to claim 5 characterized in that it contains as active ingredient a compound of formulae I or II according to patent claim 2 or a pharmaceutically acceptable salt of said compounds.

7. A pharmaceutical composition according to patent claim 5 or 6 characterized in that it contains a compound of formula I according to patent claim 3 or a pharmaceutically acceptable salt thereof.

8. A pharmaceutical composition according to one of the patent claims 5 - 7 characterized in that it contains as active ingredient a compound of claim 4 or a pharmaceutically aceeptable salt thereof.

9. A pharmaceutical composition according to one of the patent claims 5 - 8 characterized in that it contains as further ingredient a pharmaceutically acceptable carrier.